# EUROPEAN PATENT SPECIFICATION

(11) **EP 4 067 331 B1**
(45) Date of publication and mention of the grant of the patent: **29.10.2025**
(21) Application number: 20892998.4
(22) Date of filing: 26.11.2020
(51) Int. Cl.: C07C 51/02, C07C 59/66, C07C 51/09, C07C 51/43, C07C 67/31, C07C 69/712

(54) **PREPARATION METHOD FOR 2,2'-BIS(CARBOXYMETHOXY)-1,1'-BINAPHTHYL**
VERFAHREN ZUR HERSTELLUNG VON 2,2'-BIS(CARBOXYMETHOXY)-1,1'-BINAPHTHYL
PROCÉDÉ DE PRÉPARATION DE 2,2'-BIS(CARBOXYMÉTHOXY)-1,1'-BINAPHTYLE

(30) Priority: 29.11.2019 JP 2019216303
(43) Date of publication of application: 05.10.2022
(73) Proprietor: Honshu Chemical Industry Co., Ltd., Tokyo 103-0027 (JP)
(72) Inventor: SAKUMA, Daichi, Wakayama-shi, Wakayama 641-0007 (JP); SUTO, Takeru, Wakayama-shi, Wakayama 641-0007 (JP)
(74) Representative: Grünecker Patent- und Rechtsanwälte PartG mbB
(86) International application number: PCT/JP2020/044036
(87) International publication number: WO 2021/107015

(56) References cited:
- EP-A1- 3 967 677
- EP-A1- 4 067 333
- WO-A1-2019/230685
- WO-A1-2020/226114
- JP-A- 2005 194 243
- KUMARESH GHOSH ET AL: "( rac )-1,1'-Binaphthyl-Based Simple Receptors Designed for Fluorometric Discrimination of Maleic and Fumaric Acids", JOURNAL OF PHYSICAL CHEMISTRY PART B, vol. 115, no. 26, 7 July 2011 (2011-07-07), US, pages 8597 - 8608, XP055756971, ISSN: 1520-6106, DOI: 10.1021/jp202304k
- EMA, TADASHI ET AL.: "Synthesis and evaluation of chiral selectors with multiple hydrogen-bonding sites in the macrocyclic cavities", J. ORG. CHEM., vol. 75, no. 13, 2010, pages 4492 - 4500, XP055807637, ISSN: 0022- 3263, DOI: 10.1021/jo1006587
- GHOSH, KUMARESH ET AL.: "rac)-l, 1'-Binaphthyl- based simple receptors designed for fluorometric discrimination of maleic and fumaric acid", THE JOURNAL OF PHYSICAL CHEMISTRY B, vol. 115, no. 26, 2011, pages 8597 - 8608, XP055756971, ISSN: 1520-5207, DOI: 10.1021/jp202304k
- JI, FENGYING ET AL.: "Synthesis of 1,1'-bi-2,2'- naphthol diethers", ZHONGSHAN DAXUE XUEBAO, ZIRAN KEXUEBAN, vol. 35, no. 3, 1996, pages 120 - 123, XP055756968, ISSN: 0529-6579
- COLUCCINI, CARMINE ET AL.: "Locked chromophores as CD and NMR probes for the helical conformation of tetraamidic macrocycles", ORGANIC & BIOMOLECULAR CHEMISTRY, vol. 8, no. 8, 2010, pages 1807 - 1815, XP055756979, ISSN: 1477-0520, DOI: 10.1039/b924400j

## Description

### Technical Field

The present invention relates to a method for producing 2,2'-bis(carboxymethoxy)-1,1'-binaphthyl.

### Background Art

Polyester resins and polyester carbonate resins produced using dicarboxylic acid components having a binaphthalene skeleton as polymerization components have excellent optical properties such as high refractive indices and low birefringence and have high levels of heat resistance, and thus have recently been expected to be materials for optical members such as optical disks, transparent conductive substrates, and optical filters. In particular, resins produced using 2,2'-bis(carboxymethoxy)-1,1'-binaphthyl, which has a chemical structure represented by a chemical formula below, as a polymerization component have been attracting attention for their particularly excellent optical properties (see, for example, PTLs 1 to 3). 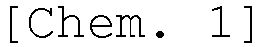 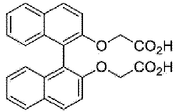 Known methods for producing 2,2'-bis(carboxymethoxy)-1,1'-binaphthyl represented by the chemical formula above include reaction of 1,1'-binaphthalene-2,2'-diol with a halogenated acetate ester, as shown by a reaction formula below, and hydrolysis of a diester obtained by the reaction (see, for example, PTL 4). Ghosh, K. et al. discloses a method for producing 2,2'-bis(carboxymethoxy)-1,1'-binaphthyl (Journal of Physical Chemistry B, 2011, 115, 8597-8608).

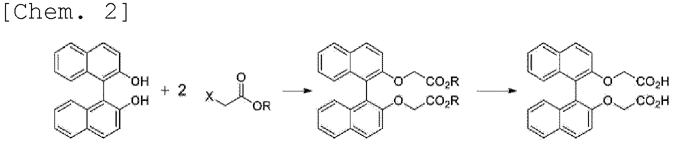

There has been a problem in that if a reaction solution contains an alcohol when acidified after the above hydrolysis reaction, a carboxylic acid of 2,2'-bis(carboxymethoxy)-1,1'-binaphthyl reacts with the alcohol to produce an ester, resulting in decreases in the yield and purity of the target. To avoid this problem, a method has been used in which after the hydrolysis reaction, a reaction solvent containing the ester-derived alcohol is distilled off from the reaction system under reduced pressure, and the reaction solution is then acidified. However, the target obtained by this method is not sufficiently pure and needs to be further purified. In addition, when the reaction solvent is distilled off from the reaction system under reduced pressure, it is necessary to add the reaction solvent decreased by the distillation under reduced pressure later or to use the reaction solvent in a large amount in advance from the start of the reaction, and, furthermore, a solvent obtained by the distillation under reduced pressure contains the ester-derived alcohol and thus cannot be reused and has to be discarded. From an industrial point of view, the generation of such waste needs to be avoided.

Under these circumstances, there has been a need for an efficient production method that can provide the target 2,2'-bis(carboxymethoxy)-1,1'-binaphthyl in high yield and high purity without distilling off the reaction solvent containing the ester-derived alcohol from the reaction system under reduced pressure after the hydrolysis reaction.

### Citation List

### Patent Literature

PTL 1: Japanese Unexamined Patent Application Publication No. 2018-002893
PTL 2: Japanese Unexamined Patent Application Publication No. 2018-002894
PTL 3: Japanese Unexamined Patent Application Publication No. 2018-002895
PTL 4: Japanese Unexamined Patent Application Publication No. 2008-024650

### Summary of Invention

### Technical Problem

The present invention has been made in view of the foregoing circumstances, and an object thereof is to provide a production method that can provide highly pure 2,2'-bis(carboxymethoxy)-1,1'-binaphthyl with good production efficiency.

### Solution to Problem

To achieve the above object, the present inventors have conducted intensive studies and found that the target can be obtained with good production efficiency and in high purity by performing a separation step of separating a metal salt of 2,2'-bis(carboxymethoxy)-1,1'-binaphthyl from a reaction mixture by solid-liquid separation, thereby completing the present invention.

The present invention is as follows.
1. A method for producing 2,2'-bis(carboxymethoxy)-1,1'-binaphthyl represented by formula (3) below, including a separation step of separating a metal salt of 2,2'-bis(carboxymethoxy)-1,1'-binaphthyl, the metal salt being represented by formula (2) below, from a reaction mixture by solid-liquid separation, the separation step comprising filtration of the metal salt and removal of a mother liquor adhering to the metal salt wherein a 2,2'-bis(alkoxycarbonylmethoxy)-1,1'-binaphthyl represented by formula (1) below is used as a starting material. 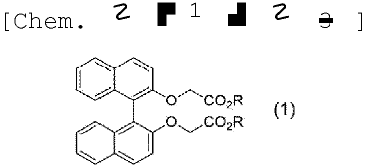 (In the formula, each R independently represents an alkyl group having 1 to 8 carbon atoms.) 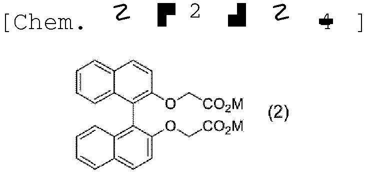 (In the formula, each M independently represents sodium or potassium.) 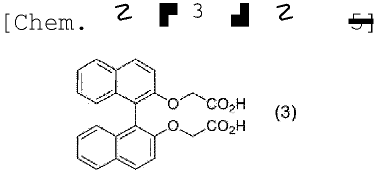 further comprising a reaction step of reacting 1,1'-binaphthalene-2,2'-diol with a halogenated acetate ester represented by formula (4) below to obtain the 2,2'-bis(alkoxycarbonylmethoxy)- 1,1'-binaphthyl represented by formula (1), [Chem. 4]

   XCH₂COOR (4)

   where X represents a halogen atom, and R represents an alkyl group having 1 to 8 carbon atoms, and wherein the synthesis of the diester (1) is performed in the presence of a reaction solvent selected from a linear or cyclic ketone solvent having 5 to 8 carbon atoms or a combination of two or more selected from a linear or cyclic ketone solvent having 5 to 8 carbon atoms and linear nitrile solvents having 2 to 6 carbon atoms.
2. The method for producing 2,2'-bis(carboxymethoxy)-1,1'-binaphthyl according to 1., including a decomposition step in which the metal salt obtained in the separation step and an acid are used.
3. The method for producing 2,2'-bis(carboxymethoxy)-1,1'-binaphthyl according to 2. wherein the reaction solvent in the decomposition step is a linear or cyclic ketone having 5 to 8 carbon atoms selected from diethyl ketone, methyl isobutyl ketone, methyl amyl ketone, 2-octanone, cyclopentanone, and cyclohexanone.

### Advantageous Effects of Invention

According to the present invention, the problem of the traditional hydrolysis reaction in that the ester-derived alcohol and the target react with each other to produce an ester, resulting in a decrease in purity of the target, is overcome, and highly pure 2,2'-bis(carboxymethoxy)-1,1'-binaphthyl can be obtained.

Moreover, the present invention does not need the method which has been employed to solve the above problem and in which the reaction solvent containing the ester-derived alcohol is distilled off from the reaction system under reduced pressure, and thus the amount of reaction solvent used can be reduced, the time, the cost of fuel and light, the cost of materials, and the like required for the entire production of the highly pure target can be reduced, and, in addition, the generation of useless waste can be suppressed, which are advantageous also from an industrial point of view.

That is, the provision of the production method of the present invention is very useful in industrial production of resin raw materials and the like because the target can be obtained with good production efficiency and in high purity. Description of Embodiments

Hereinafter, the present invention will be described in detail.

The production method of the present invention is a method represented by the following reaction formula and includes a separation step of separating a metal salt of 2,2'-bis(carboxymethoxy)-1,1'-binaphthyl, the metal salt being represented by formula (2) below, from a reaction mixture by solid-liquid separation. Hereinafter, a 2,2'-bis(alkoxycarbonylmethoxy)-1,1'-binaphthyl represented by formula (1) below is referred to as a "diester (1)", a metal salt of 2,2'-bis(carboxymethoxy)-1,1'-binaphthyl, the metal salt being represented by formula (2) below, as a "metal salt (2)", and 2,2'-bis(carboxymethoxy)-1,1'-binaphthyl represented by formula (3) below as "Target A". 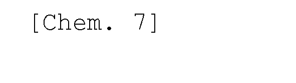 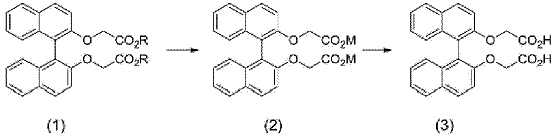 (In the formulae, each R independently represents an alkyl group having 1 to 8 carbon atoms, and each M independently represents sodium or potassium.)

### <Reaction step of obtaining diester (1)>

The "diester (1)" in the present invention can be obtained by reacting 1,1'-binaphthalene-2,2'-diol with a halogenated acetate ester represented by formula (4) below (hereinafter also referred to as the "ester (4)"), as shown by the reaction formula below. 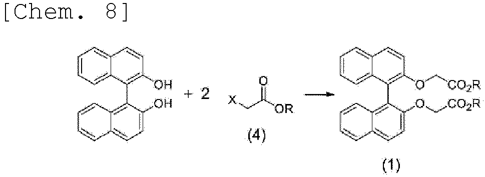 (In the formula, each R independently represents an alkyl group having 1 to 8 carbon atoms.)

### (Ester (4))

"R" in the formula of the ester (4) used to synthesize the diester (1) is an alkyl group having 1 to 8 carbon atoms, and specific examples include linear alkyl groups such as a methyl group, an ethyl group, and a n-propyl group, and branched alkyl groups in which carbon bonded to an oxygen atom is primary or secondary carbon, such as an i-propyl group and an i-butyl group. Among them, linear alkyl groups are preferred. Likewise, "X" in the formula is a halogen atom, preferably a chlorine atom, a bromine atom, or an iodine atom.

In the synthesis of the diester (1), two or more esters (4) having different alkyl ester moieties may be used in combination, but for simple and easy purification, it is preferable to use a single ester (4).

The amount of the ester (4) used is not particularly limited as long as the molar ratio of the ester (4) to 1,1'-binaphthalene-2,2'-diol is more than or equal to a theoretical value (2.0), and the ester (4) is typically used in an amount of 2 mol or more, preferably in an amount of 2.1 to 3.0 mol, more preferably in an amount of 2.2 to 2.8 mol.

### (Reaction solvent)

The synthesis of the diester (1) may be performed without a reaction solvent but is preferably performed using a reaction solvent for reasons of, for example, ease of operation in industrial production and improvement in reaction rate. The reaction solvent is not particularly limited as long as it is not distilled out of a reaction vessel at a reaction temperature and is inactive in the reaction, and examples include linear or cyclic ketone solvents having 5 to 8 carbon atoms, such as diethyl ketone, methyl isobutyl ketone, methyl amyl ketone, 2-octanone, cyclopentanone, and cyclohexanone, and linear nitrile solvents having 2 to 6 carbon atoms, such as acetonitrile and propanenitrile. These reaction solvents may be used alone or may be used in an appropriate combination of two or more to adjust polarity. In particular, methyl isobutyl ketone and acetonitrile are preferred. The amount of reaction solvent used is preferably in the range of 150 to 500 parts by weight, more preferably in the range of 200 to 300 parts by weight, relative to 100 parts by weight of 1,1'-binaphthalene-2,2'-diol.

### (Base)

In the synthesis of the diester (1), 1,1'-binaphthalene-2,2'-diol is preferably formed into a salt with a base before being reacted with the ester (4). The base is not particularly limited, and, for example, alkali metal carbonates such as lithium carbonate, sodium carbonate, and potassium carbonate; alkali metal hydrogen carbonates such as sodium hydrogen carbonate and potassium hydrogen carbonate; alkali metal hydroxides such as lithium hydroxide, sodium hydroxide, and potassium hydroxide; and organic bases such as triethylamine and pyridine are suitable for use. These may be used alone or as a mixture of two or more. In particular, sodium carbonate and potassium carbonate are preferred.

The amount of base used is preferably 2.0 to 2.5 mol, more preferably 2.05 to 2.15 mol, relative to 1 mol of 1,1'-binaphthalene-2,2'-diol.

### (Alkali metal iodide)

In the synthesis of the diester (1), the reaction may be carried out in the presence of an alkali metal iodide. Specific examples of the alkali metal iodide include potassium iodide, sodium iodide, cesium iodide, and lithium iodide. These may be used alone or as a mixture of two or more.

The amount of alkali metal iodide used is preferably in the range of 1 to 25 mol%, more preferably in the range of 2 to 15 mol%, still more preferably in the range of 2.5 to 10 mol%, particularly preferably in the range of 3 to 5 mol%, relative to 1 mol of 1,1'-binaphthalene-2,2'-diol.

### (Reaction temperature and pressure)

The reaction temperature is typically 50°C to 150°C, preferably in the range of 70°C to 130°C, more preferably in the range of 90°C to 110°C. A high reaction temperature is not preferred because the yield decreases due to, for example, hydrolysis of the resulting diester (1), and a low reaction temperature is not preferred because the reaction rate slows down. The reaction is typically carried out under normal pressure, but depending on the boiling point of an organic solvent used, the reaction may be carried out under increased pressure or reduced pressure so that the reaction temperature falls within the above range.

### (Reaction endpoint)

The endpoint of the reaction can be determined by liquid chromatography or gas chromatography analysis. The endpoint of the reaction is preferably defined as the time point at which unreacted 1,1'-binaphthalene-2,2'-diol has disappeared and the increase of the diester (1) is no longer observed. Although the reaction time varies depending on the reaction conditions such as reaction temperature, the reaction is typically completed in about 1 to 30 hours.

After completion of the reaction, water is added to the reaction solution, the mixture is stirred, and the resultant is then left to stand to separate an aqueous layer. This water washing operation is performed twice or more, whereby the inorganic salt in the reaction solution can be removed. The amount of water used in one water washing operation is preferably in the range of 150 to 600 parts by weight, more preferably in the range of 200 to 400 parts by weight, relative to 100 parts by weight of 1,1'-binaphthalene-2,2'-diol used, and the temperature in the operation is preferably in the range of 60°C to 100°C, more preferably in the range of 70°C to 90°C. The stirring may be performed in any manner as long as an oil layer is sufficiently brought into contact with an aqueous layer, and although the time required varies depending on the apparatus, about 30 minutes usually suffices.

### <Method of synthesizing metal salt (2)>

### (Reaction solvent)

For the synthesis of the metal salt (2) in the present invention, for example, a solution that has been through the water washing operation after completion of the synthesis reaction of the diester (1) can be used. When the diester (1) that has been purified is used, it is preferable to use, as a reaction solvent, a mixed solvent of an organic solvent and water. Specific examples of the organic solvent used include linear or cyclic ketone solvents having 5 to 8 carbon atoms, such as diethyl ketone, methyl isobutyl ketone, methyl amyl ketone, 2-octanone, cyclopentanone, and cyclohexanone, and linear nitrile solvents having 2 to 6 carbon atoms, such as acetonitrile and propanenitrile. The amount of organic solvent used is, for example, preferably 100 to 600 parts by weight, more preferably 130 to 400 parts by weight, relative to 100 parts by weight of the diester
(1). The amount of water used is preferably 10 to 200 parts by weight, more preferably 20 to 150 parts by weight, relative to 100 parts by weight of the diester (1).

### (Base)

To convert the diester (1) into the metal salt (2), a base is used. Specific examples of the base used include alkali metal hydroxides such as sodium hydroxide and potassium hydroxide, and by using such a base, an alkali metal salt can be obtained as the metal salt (2). The base may be used as a solid or in the form of an aqueous solution. The concentration of the base used in the form of an aqueous solution is preferably 10 to 60 wt%, more preferably 20 to 50 wt%.

The amount of the base used is preferably 2.0 to 6.0 mol, more preferably 2.5 to 4.0 mol, relative to 1 mol of the diester (1).

### (Reaction temperature)

The reaction temperature is typically 30°C to 100°C, preferably in the range of 50°C to 90°C, more preferably in the range of 60°C to 80°C, and it is preferable to add or drop the above base or an aqueous solution thereof while maintain this temperature.

The reaction is typically completed in about 1 to 10 hours.

### <Step of separating metal salt (2)>

The production method of the present invention includes a separation step of separating the metal salt (2) from a reaction mixture by solid-liquid separation.

After completion of the reaction for obtaining the metal salt (2), the reaction solution is preferably cooled. The rate of cooling is preferably 5°C to 15°C per hour, more preferably 7°C to 12°C per hour. The final cooling temperature is preferably 20°C to 60°C, more preferably 25°C to 35°C. After the reaction solution is cooled, the precipitated metal salt (2) is filtered, whereby the metal salt (2) is obtained.

The mother liquor adhering to the metal salt (2) obtained is removed as much as possible. For this purpose, the metal salt (2) obtained may be washed with a solvent. The washing may be performed using a mixed solvent of, for example, water and the organic solvent used in the synthesis of the metal salt (2) or using water and the organic solvent separately, for example, in a manner that washing with water is first performed and washing with the organic solvent is then performed. The amount of the mixed solvent used is preferably in the range of 10 to 200 parts by weight, more preferably in the range of 40 to 90 parts by weight, relative to 100 parts by weight of the diester (1).

### <Decomposition step for obtaining Target A>

Target A in the present invention can be obtained through a decomposition step in which the metal salt (2) and an acid are used. Specific examples of the acid used in the decomposition step include hydrogen chloride, hydrogen bromide, inorganic acids such as sulfuric acid, and organic acids such as p-toluenesulfonic acid, methanesulfonic acid, and trifluoromethanesulfonic acid. The amount of the acid used is preferably 2.2 to 4.0 mol, more preferably 2.5 to 3.0 mol, relative to 1 mol of the diester (1).

When concentrated hydrochloric acid is used as the acid, the amount thereof in terms of hydrogen chloride is preferably 2.2 to 4.0 mol, more preferably 2.5 to 3.0 mol, relative to 1 mol of the diester (1).

Specific examples of a reaction solvent in the decomposition step include linear or cyclic ketone solvents having 5 to 8 carbon atoms, such as diethyl ketone, methyl isobutyl ketone, methyl amyl ketone, 2-octanone, cyclopentanone, and cyclohexanone. The amount of reaction solvent used is preferably 250 to 1050 parts by weight, more preferably 350 to 900 parts by weight, relative to 100 parts by weight of the diester (1).

In addition to the above reaction solvent, water can optionally be used in combination in order, for example, to prevent precipitation of salts.

### <Step of recovering Target A>

After the decomposition step, the reaction solution is left to stand, after which an aqueous layer is extracted, and a dissolved target can be recovered from the resulting oil layer by a known method. For example, the target can be obtained by performing a water washing operation involving addition of water to the oil layer, stirring, and separation of an aqueous layer multiple times as needed, crystallizing the oil layer, and filtering the resultant. In the crystallization, the amount of solvent and the amount of water can be adjusted as needed by a distillation operation or the like in order, for example, to improve the yield.

### EXAMPLES

The present invention will now be described more specifically with reference to Examples, but it should be noted that the present invention is not limited to these Examples.

The method of analysis is as follows.

### <Method of analysis>

### 1. Determination of target concentration in reaction solution, reaction yield, and reaction endpoint

After the measurement was performed under the following conditions, the purity (%) of Targets A obtained in Examples and Comparative Examples was calculated using a liquid chromatography calibration curve of the target compound.

Measuring apparatus: high-performance liquid chromatography analyzer (manufactured by Shimadzu Corporation)
Pump: LC-20AD
Column oven: CTO-20A
Detector: SPD-20A
Column: HALO-C18
Oven temperature: 50°C
Flow rate: 0.7 ml/min
Mobile phase: (A) acetonitrile, (B) 0.1 vol% phosphoric acid solution
Gradient conditions: (A) volume % (time from start of analysis)
30% (0 min) → 100% (12 min) → 100% (15 min)
Detection wavelength: 280 nm

### <Example 1>

### · Production method 1 of present invention

In a four-necked flask, 1213 g of 1,1'-binaphthalene-2,2'-diol, 3638 g of acetonitrile, 1346 g of potassium carbonate, and 121 g of potassium iodide were placed, heated to 70°C, and stirred at this temperature for one hour. After a mixed solution of 1460 g of ethyl chloroacetate and 13 g of N-methylpyrrolidone was prepared, the mixed solution was added dropwise while maintaining the temperature of the reaction solution at 70°C to 80°C. After stirring for six hours, 3032 g of water was added, and the mixture was heated to 70°C and then left to stand to remove an aqueous layer. Subsequently, 3392 g of a 35% aqueous potassium hydroxide solution was added dropwise to the resulting oil layer while maintaining the reaction solution temperature at 70°C to 80°C.

After two hours, the reaction solution was gradually cooled and filtered at 25°C (corresponding to the separation step of the present invention) to obtain crystals. The crystals were washed with water (606 g) and then washed with acetonitrile (606 g) to obtain 2180 g of a potassium salt of 2,2'-bis(carboxymethoxy)-1,1'-binaphthyl. For the removal of a mother liquor during the filtration, centrifugal force (small centrifuge H-122, manufactured by KOKUSAN Co., Ltd.) was utilized.

In a four-necked flask, 2051 g of the obtained potassium salt (undried), 3430 g of water, and 9702 g of methyl isobutyl ketone were placed and heated to 80°C to be dissolved. Concentrated hydrochloric acid in an amount of 1207 g was added dropwise while maintaining the temperature at 80°C to 85°C, and the resultant was stirred at this temperature for 30 minutes. After standing, an aqueous layer was extracted, and a water washing operation involving addition of water to the resulting oil layer, stirring, and removal of an aqueous layer by separation was performed multiple times until the pH of the aqueous layer reached 4. Subsequently, under normal pressure, 4713 g of water and methyl isobutyl ketone was distilled out of the resulting oil layer by distillation. At 95°C midway through the distillation, seed crystals obtained by a production method known in the art were added, and precipitation of crystals was checked. After this, the solution in which crystals were precipitated was cooled to 25°C at a cooling rate of 10°C per hour and filtered, and the residue was then dried under reduced pressure to obtain 1392 g of a crystalline body of Target A (yield: 86.8%, purity: 99.9%, monoethyl ester: 0.05%).

### <Example 2>

### · Production method 2 of present invention

In a four-necked flask, 35 g of 1,1'-binaphthalene-2,2'-diol, 52.5 g of methyl isobutyl ketone, 35.5 g of potassium carbonate, and 0.7 g of potassium iodide were placed, heated to 100°C, and stirred at this temperature for two hours. After a mixed solution of 33.0 g of ethyl chloroacetate and 0.3 g of N-methylpyrrolidone was prepared, the mixed solution was added dropwise while maintaining the temperature of the reaction solution at 90°C to 100°C. After stirring for 10 hours, 140 g of water was added, and the mixture was heated to 80°C and then left to stand to remove an aqueous layer. Subsequently, 105.0 g of methyl isobutyl ketone was added to the resulting oil layer, and 30.6 g of a 48% aqueous sodium hydroxide solution was added dropwise while maintaining the reaction solution temperature at 80°C to 85°C.

After two hours, the reaction solution was gradually cooled and filtered at 25°C (corresponding to the separation step of the present invention) to obtain crystals. The crystals were found to have low solubility in water (concentration of saturated solution in water at 30°C: 3.8 wt%). The crystals were washed with water (18 g) and then washed with methyl isobutyl ketone (18 g) to obtain 109.0 g of a sodium salt of 2,2'-bis(carboxymethoxy)-1,1'-binaphthyl. For the removal of a mother liquor during the filtration, centrifugal force (small centrifuge H-122, manufactured by KOKUSAN Co., Ltd.) was utilized.

In a four-necked flask, 63.5 g of the obtained sodium salt (undried), 244.7 g of methyl isobutyl ketone, and 10.2 g of water were placed and heated to 80°C to be dissolved. Concentrated hydrochloric acid in an amount of 22.2 g was added dropwise while maintaining the temperature at 80°C to 85°C, and the resultant was stirred at this temperature for 30 minutes. After standing, an aqueous layer was extracted, and a water washing operation involving addition of water to the resulting oil layer, stirring, and removal of an aqueous layer by separation was performed multiple times until the pH of the aqueous layer reached 4. Subsequently, under normal pressure, 197.2 g of water and methyl isobutyl ketone was distilled out of the resulting oil layer by distillation. At 95°C midway through the distillation, seed crystals obtained by a production method known in the art were added, and precipitation of crystals was checked. After this, the solution in which crystals were precipitated was cooled to 25°C at a cooling rate of 10°C per hour and filtered, and the residue was then dried under reduced pressure to obtain 25.2 g of a crystalline body of Target A (yield: 87.8%, purity: 99.9%, monoethyl ester: 0.05%).

### <Comparative Example 1>

### · Production method different from production method of present invention

In a four-necked flask, 35 g of 1,1'-binaphthalene-2,2'-diol, 52.5 g of methyl isobutyl ketone, 35.5 g of potassium carbonate, and 0.7 g of potassium iodide were placed, heated to 100°C, and stirred at this temperature for two hours. After a mixed solution of 33.0 g of ethyl chloroacetate and 0.3 g of N-methylpyrrolidone was prepared, the mixed solution was added dropwise while maintaining the temperature of the reaction solution at 90°C to 100°C. After stirring for 10 hours, 140 g of water was added, and the mixture was heated to 80°C, after which an aqueous layer was removed. Subsequently, 105.0 g of methyl isobutyl ketone was added, and 30.6 g of a 48% aqueous sodium hydroxide solution was added dropwise while maintaining the reaction solution temperature at 80°C to 85°C.

After two hours, 185.5 g of methyl isobutyl ketone and 105.0 g of water were added to the reaction solution, after which 38.2 g of concentrated hydrochloric acid was added dropwise while maintaining the temperature at 80°C to 85°C, and the resultant was stirred at this temperature for 30 minutes. After standing, an aqueous layer was extracted, and a water washing operation involving addition of water to the resulting oil layer, stirring, and removal of an aqueous layer by separation was performed multiple times until the pH of the aqueous layer reached 4. Subsequently, under normal pressure, 231.6 g of water and methyl isobutyl ketone was distilled out of the resulting oil layer by distillation. At 95°C midway through the distillation, seed crystals obtained by a production method known in the art were added, and precipitation of crystals was checked. After this, the solution in which crystals were precipitated was cooled to 25°C at a cooling rate of 10°C per hour and filtered to obtain 51.5 g of a crystalline body of Target A (purity: 98.3%, monoethyl ester: 1.6%).

### (Crystallization of Target A)

In a four-necked flask, 30.0 g of the obtained crystalline body of Target A, 183.9 g of methyl isobutyl ketone, and 9.8 g of water were placed and heated to 85°C to be dissolved. Subsequently, under normal pressure, 119.6 g of water and methyl isobutyl ketone was distilled out by distillation. At 95°C midway through the distillation, seed crystals obtained by a production method known in the art were added, and precipitation of crystals was checked. After this, the crystallized solution was cooled to 25°C at a cooling rate of 10°C per hour and filtered, and the residue was then dried under reduced pressure to obtain 24.8 g of a crystalline body of Target A (yield: 86.4%, purity: 99.2%, monoethyl ester: 0.7%).

### <Comparative Example 2>

In a four-necked flask, 38 g of 1,1'-binaphthalene-2,2'-diol, 57 g of methyl isobutyl ketone, 38.5 g of potassium carbonate, and 0.76 g of potassium iodide were placed, heated to 100°C, and stirred at this temperature for two hours. After a mixed solution of 35.8 g of ethyl chloroacetate and 0.3 g of N-methylpyrrolidone was prepared, the mixed solution was added dropwise while maintaining the temperature of the reaction solution at 90°C to 100°C. After stirring for 10 hours, 152 g of water was added, and the mixture was heated to 80°C and then left to stand to remove an aqueous layer. After 114 g of methyl isobutyl ketone was added to the resulting oil layer, 33.2 g of a 48% aqueous sodium hydroxide solution was added dropwise while maintaining the temperature of the reaction solution at 80°C to 85°C.

After stirring for two hours, 152 g of methyl isobutyl ketone was added, and 152 g of methyl isobutyl ketone, ethanol, and water was distilled out under normal pressure. Water in an amount of 114 g and methyl isobutyl ketone in an amount of 201.4 g were added, concentrated hydrochloric acid in an amount of 41.5 g was added dropwise while maintaining the temperature at 80°C to 85°C, and the resultant was stirred at this temperature for 30 minutes. After standing, an aqueous layer was extracted, and a water washing operation involving addition of water to the resulting oil layer, stirring, and removal of an aqueous layer by separation was performed multiple times until the pH of the aqueous layer reached 4. Subsequently, under normal pressure, 244.1 g of water and methyl isobutyl ketone was distilled out of the resulting oil layer by distillation. At 95°C midway through the distillation, seed crystals obtained by a production method known in the art were added, and precipitation of crystals was checked. The resultant was cooled to 25°C at a cooling rate of 10°C per hour and filtered to obtain 52.9 g of crystals (undried) of Target A (purity: 99.7%, monoethyl ester: 0.20%).

### (Crystallization of Target A)

In a four-necked flask, 31.2 g of the obtained crystalline body of Target A, 197.2 g of methyl isobutyl ketone, and 10.3 g of water were placed and heated to 85°C to be dissolved. Subsequently, under normal pressure, 124.9 g of water and methyl isobutyl ketone was distilled out by distillation. At 95°C midway through the distillation, seed crystals obtained by a production method known in the art were added, and precipitation of crystals was checked. After this, the crystallized solution was cooled to 25°C at a cooling rate of 10°C per hour and filtered, and the residue was then dried under reduced pressure to obtain 27.3 g of 2,2'-bis(carboxymethoxy)-1,1'-binaphthyl (yield: 86.7%, purity: 99.9%, monoethyl ester: 0.08%).

As shown by the above Examples, the production methods of the present invention (Examples 1 and 2), each involving solid-liquid separation of the metal salt (2) from a reaction mixture, were confirmed to provide extremely high-purity crystalline bodies with little monoethyl ester formation, specifically, the purity of Target A was 99.9%, and the monoethyl ester content was 0.05%.

By contrast, in the production method (Comparative Example 1) not involving solid-liquid separation of the metal salt (2) from a reaction mixture, simply precipitating a crystalline body of Target A and performing filtration provided only a crystalline body with a monoester content of 1.6 wt% and a Target A purity of 98.3%, thus revealing that the crystallization step was essential. Moreover, although crystallization and purification by filtration were further performed, only a crystalline body with a Target A purity of 99.2% and a monoethyl ester content of 0.7% was obtained.

Furthermore, also in the production method (Comparative Example 2) in which after a hydrolysis reaction, a reaction solvent containing an ester-derived alcohol was distilled off from a reaction system under reduced pressure and a reaction solution is then acidified, simply filtering a crystalline body of Target A resulted in a monoester content of 0.2 wt%, thus revealing that the crystallization step of dissolving obtained crystals in an organic solvent was essential.

The production method of the present invention was shown to be an excellent production method that does not require reduced-pressure distillation of a reaction solvent containing an ester-derived alcohol from a reaction system and can provide an extremely high-purity target while maintaining a high yield without performing the crystallization step of dissolving obtained crystals in an organic solvent. Moreover, the present invention was confirmed to be a production method that is very advantageous in industrial production of resin raw materials and the like because the amount of solvent used can be reduced as shown in Example 2, the time, the cost of fuel and light, the cost of materials, and the like required for the entire production can be reduced, and, in addition, the generation of useless waste can be suppressed.

## Claims

1. A method for producing 2,2'-bis(carboxymethoxy)-1,1'-binaphthyl represented by formula (3) below, comprising a separation step of separating a metal salt of 2,2'-bis(carboxymethoxy)-1,1'-binaphthyl, the metal salt being represented by formula (2) below, from a reaction mixture by solid-liquid separation, the separation step comprising filtration of the metal salt and removal of a mother liquor adhering to the metal salt,
wherein a 2,2'-bis(alkoxycarbonylmethoxy)-1,1'-binaphthyl represented by formula (1) below is used as a starting material, 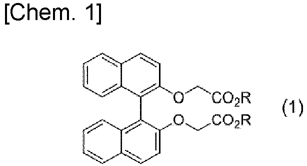 where each R independently represents an alkyl group having 1 to 8 carbon atoms, 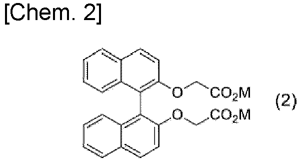 where each M independently represents sodium or potassium, 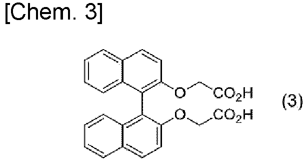 further comprising a reaction step of reacting 1,1'-binaphthalene-2,2'-diol with a halogenated acetate ester represented by formula (4) below to obtain the 2,2'-bis(alkoxycarbonylmethoxy)-1,1'-binaphthyl represented by formula (1),
[Chem. 4]
XCH₂COOR (4)
where X represents a halogen atom, and R represents an alkyl group having 1 to 8 carbon atoms, and wherein the synthesis of the diester (1) is performed in the presence of a reaction solvent selected from a linear or cyclic ketone solvent having 5 to 8 carbon atoms or a combination of two or more selected from a linear or cyclic ketone solvent having 5 to 8 carbon atoms and linear nitrile solvents having 2 to 6 carbon atoms.

2. The method for producing 2,2'-bis(carboxymethoxy)-1,1'-binaphthyl according to Claim 1, comprising a decomposition step in which the metal salt obtained in the separation step and an acid are used.

3. The method for producing 2,2'-bis(carboxymethoxy)-1,1'-binaphthyl according to Claim 2, wherein the reaction solvent in the decomposition step is a linear or cyclic ketone having 5 to 8 carbon atoms selected from diethyl ketone, methyl isobutyl ketone, methyl amyl ketone, 2-octanone, cyclopentanone, and cyclohexanone.

## Patentansprüche

1. Verfahren zur Herstellung von 2,2'-Bis(carboxymethoxy)-1,1'-binaphthyl, dargestellt durch die nachstehende Formel (3), umfassend einen Trennungsschritt zum Abtrennen eines Metallsalzes von 2,2'-Bis(carboxymethoxy)-1,1'-binaphthyl, wobei das Metallsalz durch die nachstehende Formel (2) dargestellt wird, aus einem Reaktionsgemisch durch Fest-Flüssig-Trennung, wobei der Trennungsschritt das Filtrieren des Metallsalzes und das Entfernen einer an dem Metallsalz haftenden Mutterlauge umfasst,
wobei ein 2,2'-Bis(alkoxycarbonylmethoxy)-1,1'-binaphthyl der folgenden Formel (1) als Ausgangsmaterial verwendet wird: 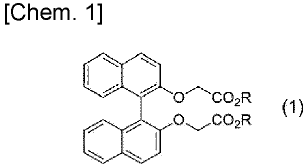 wobei jedes R unabhängig voneinander eine Alkylgruppe mit 1 bis 8 Kohlenstoffatomen darstellt, 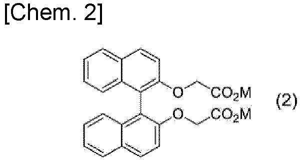 wobei jedes M unabhängig Natrium oder Kalium darstellt, 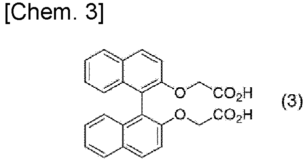 ferner umfassend einen Reaktionsschritt des Umsetzens von 1,1'-Binaphthyl-2.2'-diol mit einem halogenierten Acetatester, dargestellt durch die nachstehende Formel (4), um das 2,2'-Bis(alkoxycarbonylmethoxy)-1,1'-binaphthyl, dargestellt durch Formel (1) zu erhalten,
[Chem. 4]
XCH₂COOR (4)
wobei X ein Halogenatom darstellt, und R eine Alkylgruppe mit 1 bis 8 Kohlenstoffatomen darstellt, und wobei die Synthese des Diesters (1) in Gegenwart eines Reaktionslösungsmittels durchgeführt wird, ausgewählt aus einem linearen oder cyclischen Ketonlösungsmittel mit 5 bis 8 Kohlenstoffatomen, oder einer Kombination aus zwei oder mehr Lösungsmitteln, ausgewählt aus einem linearen oder cyclischen Ketonlösungsmitteln mit 5 bis 8 Kohlenstoffatomen und linearen Nitrillösungsmitteln mit 2 bis 6 Kohlenstoffatomen.

2. Verfahren zur Herstellung von 2,2'-Bis(carboxymethoxy)-1,1'-binaphthyl nach Anspruch 1, umfassend einen Zersetzungsschritt, in dem das in dem Trennungsschritt erhaltene Metallsalz und eine Säure verwendet werden.

3. Verfahren zur Herstellung von 2,2'-Bis(carboxymethoxy)-1,1'-binaphthyl nach Anspruch 2, wobei das Reaktionslösungsmittel in dem Zersetzungsschritt ein lineares oder cyclisches Keton mit 5 bis 8 Kohlenstoffatomen ist, ausgewählt aus Diethylketon, Methylisobutylketon, Methylamylketon, 2-Octanon, Cyclopentanon und Cyclohexanon.

## Revendications

1. Procédé de production de 2,2'-bis(carboxyméthoxy)-1,1'-binaphtyle représenté par la formule (3) ci-dessous, comprenant une étape de séparation consistant à séparer un sel métallique du 2,2'-bis(carboxyméthoxy)-1,1'-binaphtyle, le sel métallique étant représenté par la formule (2) ci-dessous, à partir d'un mélange réactionnel par séparation solide-liquide, l'étape de séparation comprenant la filtration du sel métallique et l'élimination d'une liqueur mère adhérant au sel métallique,
dans lequel un 2,2'-bis(alcoxycarbonylméthoxy)-1,1'-binaphtyle représenté par la formule (1) ci-dessous est utilisé comme matériau de départ, 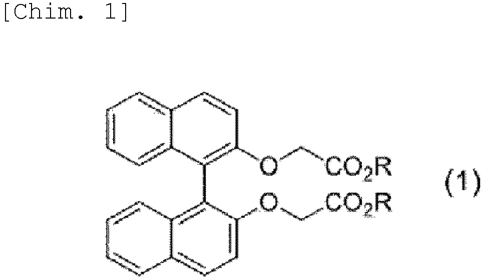 où chaque R représente indépendamment un groupe alkyle comportant de 1 à 8 atomes de carbone, 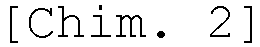 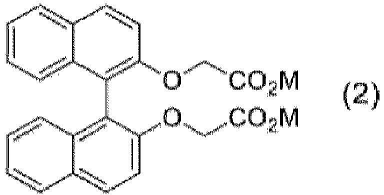 où chaque M représente indépendamment un sodium ou un potassium, 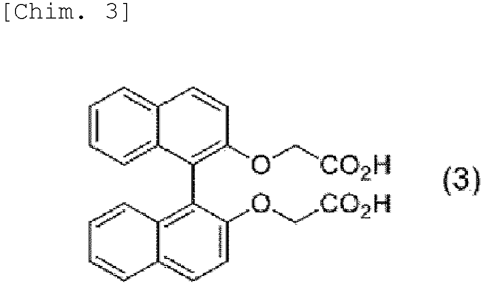 comprenant en outre une étape de réaction consistant à faire réagir du 1,1'-binaphtalène-2,2'-diol avec un ester d'acétate halogéné représenté par la formule (4) ci-dessous pour obtenir le 2,2'-bis(alcoxycarbonylméthoxy)-1,1'-binaphtyle représenté par la formule (1),
[Chim. 4]
XCH₂COOR (4)
où X représente un atome halogène, et R représente un groupe alkyle comportant de 1 à 8 atomes de carbone, et dans lequel la synthèse du diester (1) est réalisée en présence d'un solvant de réaction sélectionné parmi un solvant cétonique linéaire ou cyclique comportant de 5 à 8 atomes de carbone et une combinaison de deux ou plusieurs solvants sélectionnés parmi un solvant cétonique linéaire ou cyclique comportant de 5 à 8 atomes de carbone et des solvants nitriles linéaires comportant de 2 à 6 atomes de carbone.

2. Procédé de production de 2,2'-bis(carboxyméthoxy)-1,1'-binaphtyle selon la revendication 1, comprenant une étape de décomposition dans laquelle sont utilisés le sel métallique obtenu à l'étape de séparation et un acide.

3. Procédé de production de 2,2'-bis(carboxyméthoxy)-1,1'-binaphtyle selon la revendication 2, dans lequel le solvant de réaction à l'étape de décomposition est une cétone linéaire ou cyclique comportant de 5 à 8 atomes de carbone sélectionnée parmi la diéthylcétone, la méthylisobutylcétone, la méthylamylcétone, la 2-octanone, la cyclopentanone et la cyclohexanone.
